# NEW EUROPEAN PATENT SPECIFICATION

(11) **EP 3 440 167 B2**
(45) Date of publication and mention of the opposition decision: **04.12.2024**
(45) Mention of the grant of the patent: 13.10.2021
(21) Application number: 17709700.3
(22) Date of filing: 10.03.2017
(51) Int. Cl.: C11B 9/00, C11D 3/50

(54) **FRAGRANCED COMPOSITIONS AND THEIR USE**
DUFTSTOFFZUSAMMENSETZUNGEN UND DEREN VERWENDUNG
COMPOSITIONS DE PARFUM ET LEUR UTILISATION

(30) Priority: 04.04.2016 EP 16163620
(43) Date of publication of application: 13.02.2019
(73) Proprietor: Unilever IP Holdings B.V., 3013 AL Rotterdam (NL); Unilever Global IP Limited, Wirral, Merseyside CH62 4ZD (GB)
(72) Inventor: JONES, Craig, Warren, Bebington Wirral Merseyside CH63 3JW (GB); PARKER, Andrew, Philip, Bebington Wirral Merseyside CH63 3JW (GB)
(74) Representative: Hardy, Susan Margaret
(86) International application number: PCT/EP2017/055745
(87) International publication number: WO 2017/174296

(56) References cited:
- EP-A1- 0 965 326
- WO-A1-2007/014152
- WO-A1-2011/036048
- WO-A1-2015/032885
- WO-A1-2015/161860
- WO-A2-2012/012385
- US-A- 4 515 705
- US-A1- 2004 033 912
- DATABASE WPI Week 201635, Derwent World Patents Index; AN 2016-11343A, XP002768720
- DATABASE WPI Week 201214, Derwent World Patents Index; AN 2011-L56916, XP002760073

## Description

The present invention relates to compositions that provide a delayed fragrance release and to use of the compositions. The compositions are home care compositions.

Compositions used in home care (for example, surface cleaning) and personal care (for example, skin lotions) typically contain a fragrance, such that during and often for at least a short time after application the user experiences an aroma. This can serve to mask unpleasant odours, enhance the user experience, and increase user perception of cleanliness. The fragrance may be the result of a single aroma compound, or a blend of aroma compounds.

A vast number of suitable aroma compounds are known and used in the art. These often volatilise into the headspace of the composition container before use (such that a burst of fragrance is experienced on opening the container) and into the surrounding air when the product is used.

Over the course of the time, the aroma compounds dissipate and the discernible aroma fades. The perception of this process may be hastened as the user becomes accustomed to the fragrance: a person is less likely to perceive a weak residual fragrance because the initial identical fragrance was much stronger.

The invention is based on the inventors' insight that low levels of ozone (a pollutant present at low levels in the atmosphere of homes and offices) may be exploited to generate an aroma compound in the period after use. This nascent aroma compound may reinforce content of the same aroma compound (to boost the composition scent) or may be a different aroma compound to those present in the composition (to provide a new scent).

Accordingly, in a first aspect, the invention provides a composition according to claim 1.

It will be appreciated that the aroma compound resulting from ozonolysis typically has a carbonyl group, although further reaction of the nascent aroma compound may occur. As the level of ozone in the atmosphere is typically low, this reduces competitive reactions, ensuring more efficient release of the aroma compound.

Preferably, the remainder of the composition does not contain more than 0.5 wt% compounds having an iodine value greater than 10.

The composition is a homecare surface cleaner.

Ozonolysis of the unsaturated bond in the pro-fragrance releases cyclocitral.

The pro-fragrance is present in the composition at 0.1 to 10 wt%, preferably 0.1 to 1 wt% of the composition.

The pro-fragrance may be encapsulated within a suitable shell. Suitable shell materials include melamine formaldehyde, polyurea, and polyacrylate, and mixtures thereof. Other suitable encapsulate materials will be apparent one skilled in the art.

Furthermore, the composition may include a deposition aid to increase deposition / retention on the surface being treated. Suitably, the deposition aid is at least partially attached / integral to the encapsulate shell. Suitable deposition aids and methods for their use are known in the art.

In some embodiments, the composition comprises a hydrophobic oil having an octanol/water partition co-efficient (log P) greater than 3.

The composition may comprise one or more aroma compounds. These may be the same as the aroma compound released by ozonolysis, or different. In some embodiments, the composition comprises an aroma compound having an iodine value of about 0. According to a second aspect of the invention there is provided the use of a composition according to the first aspect for treating a surface whereby after application of the composition to the surface the β-ionone reacts with ozone in the atmosphere to produce fragrance changing levels of cyclocitral.

### Pro-fragrance Compound

The pro-fragrance compound comprises an unsaturated bond that is susceptible to ozonolysis. Once this bond is ozonolysed, an aroma compound is released. Accordingly, the pro-fragrance compound has an iodine value greater than 100. The pro-fragrance compound of the present invention is β-ionone, which has an iodine value of around 260.

It will be appreciated that the pro-fragrance compound itself is an aroma compound.

In this way, the pro-fragrance compound itself lends a first fragrance, then after ozonolysis, the nascent aroma compound is detected and the recipient experiences a second fragrance. As a recipient may already have some acclimatisation to the first fragrance, the second fragrance may be more perceptible as different olfactory receptors are stimulated. This improves the user experience and contributes to the perception of freshness.

### Aroma Compound

An aroma compound is a volatile compound that is detectable by the olfactory system. Aroma compounds are also known as odorants, aromas, and fragrances, and sometimes as favours (when both taste and smell are affected). They may be natural or synthetic. It will be appreciated that scents may be perceived differently by different recipients. Where the scent of an aroma compound is described, this is to help illustrate the invention, and is based on a commonly accepted description of the scent.

The present invention describes pro-fragrances that ozonolyse to release aroma compounds. The pro-fragrance compound may itself be an aroma compound. Of course, other aroma compounds that are not susceptible to, or produced as a result of, ozonolysis. Where there is ambiguity, the aroma compound produced as a result of ozonolysis of a pro-fragrance is referred to as a nascent aroma compound.

### Pro-fragrance

The pro-fragrance compound according to the present invention is β-ionone, which has a rose scent. On ozonolysis, the disubstituted double bond cleaves to release a cyclocitral aroma compound which may have a minty / fresh green odour. The composition as used may have a floral aroma which yields to a minty fresh odour as β-cyclocitral is generated.

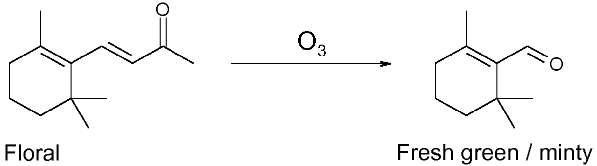

### Nascent Aroma Compound

The nascent aroma compound is produced by ozonolysis of the pro-fragrance. For simplicity, it may also be referred to as the aroma compound. The nascent aroma compound is as described above, namely cyclocitral.

### Iodine Value

This may also be referred to the iodine adsorption value" or "iodine index". It measures the degree of, and to an extent the reactivity of, unsaturation in a compound. Values are given in units of grams of iodine consumed by reaction by 100 grams of the substance. For example, if 100 g of a substance consumes 4 grams of iodine, the iodine value is 4. Methods for determination of iodine value are well known and practised in the art.

### Rate Constant

Suitably, the pro-fragrance has a second-order rate constant with ozone of greater than 2000M⁻¹s⁻¹.

### Composition Types

The composition is a homecare composition. It will be appreciated that, suitably, the composition is a composition that is applied to a substrate.

The composition is a hard surface cleaner (multi-purpose, surface, window, floor and the like). The composition may be a product that is normally used neat (for example, a sanitising spray such as those used to clean surfaces in kitchens and bathrooms) or a product that is diluted (for example, a multipurpose cleaner). The composition may be non-rinse formulation, or a formulation for which a rinse is advised. Preferably, the composition is intended to be used without a rinse.

Accordingly, the composition comprises a surfactant. The total surfactant content is 2 wt% to 15 wt% of the composition.

### Fixative

The composition may include a fixative. Fixatives may be included in fragranced compositions and are substances which amplify the intensity and lasting qualities of aroma compounds in the fragrance. The fixative suitably reduced the rate of perfume loss from the surface, such that aroma compounds are released over a longer period of time after application.

Suitably, the fixative is a hydrophobic oil having an octanol/water partition co-efficient (log P) greater than 3, preferably greater than 5. The hydrophobic oil suitably has a Hildebrand's solubility parameter (δ_{H}I_{LD}) (Hildebrand, J.H. & Scott, R.L. The Solubility of Nonelectrolytes, 3rd edition, Reinhold, New York, 1950) of less than 20 MPa^{1/2}, and preferably greater than 14 MPa^{1/2}.

The fixative oil preferably does not compete with the perfume. Suitably, the fixative is odourless, of low odour, or of neutral or inoffensive odour. Suitably, the fixative contains no unsaturated bonds.

Suitably, the fixture is a hydrophobic non-ionic oil which is liquid at skin temperature (typically about 35 °C under normal conditions). Suitably, hydrophobic oil is non-volatile, preferably having a vapour pressure of less than about 0.01 mm Hg.

The hydrophobic oil may comprise an ester and / or an ether and / or a polyether. For example, the hydrophobic oil may comprise propylene glycol dicaprate/dicaprylate, dioctyl adipate, isopropyl myristate, poly(oxyethylene)(10) cetyl ether, poly(oxyethylene)(20) sorbitan monolaurate, dibutyl phthalate, acetyl tributyl citrate, or any mixture thereof.

In some cases, the composition may contain hydrophobic oil up to 30 wt% with respect to the surfactant concentration. Preferably, the total hydrophobic oil is 3 wt% or less of the total composition. In some embodiments, the composition comprises about 10 wt% surfactant and less than 3wt% fixative.

It will be understood that it is desirable that the fixative does not react to a significant extent with ozone. Accordingly, suitably any fixative used has an iodine value of <5, preferably <3, more preferable <1, most preferably about 0.

### Example 1

A confidential consumer test for a home care composition was carried out using β-ionone as a pro-fragrance compound (Test formulation) and a control formulation. The home care composition was a general purpose floor cleaner.

A sequential monadic home use test in four cities in Northern Italy (Padova, Milan, Turin, and Bologna) was conducted with 480 consumers during February 2015. The consumers were recruited against the following criteria: 20-65 year old housewives, responsible for cleaning the house, brand decision makers for the purchase of multi-purpose cleaners, and use multi-purpose cleaners at least twice per week. The consumers were informed that the test was confidential.

The consumers were split into two cells: Cell A started with the Control formulation for two weeks prior to switching to the Test formulation; Cell B started with the Test formulation for two weeks prior to switching to the Control formulation. It should be noted that the first product was removed by the study co-ordinator before placing the second product with the consumer. The products were placed with the consumers in a non-branded context. However, typical use guidelines were given such as the recommended dose of 12 g of formulation added to 1 L of water, which the consumer could choose to apply or not depending upon their current habits.

The two fragrances were hedonically balanced to fit within the citrus family, to fit with the expectations of consumers that use floor cleaning products. Table 1 contains the two floor cleaner formulations used in the consumer study.

**Table 1**

| **Ingredient** | **Trade Name** | **% Activity as Supplied** | **% w/w. Control** | **% w/w. Test Formulation** |
|---|---|---|---|---|
| Demineralized water | | 100 | To 100 | To 100 |
| 1,2benxoisothiazolinone^{a} | Nipacide BIT 20 | 20 | 0.016 | 0.016 |
| Ethoxylated alcohol C10-C11 10 EO D^{b} | Neodol 91-8 | 100 | 2.00 | 2.00 |
| Coco fatty acids (saturated)^{c} | Prifac 7909 | 100 | 0.085 | 0.085 |
| Citric Acid | | 50 | 0.20 | 0.20 |
| Sodium Hydroxide | | 50 | 0.085 | 0.085 |
| CI 19140^{e} | Tartrazine Y | 0.1 | 0.0004 | 0.0004 |
| Perfume 1^{f} | AUBADE 1 | 100 | 0.0 | 0.30 |
| Perfume 2^{g} | AUBADE 2 | 100 | 0.30 | 0.0 |
| ^{a}Ex. Clariant; ^{b}Ex. Sasol; ^{c}Ex. Croda; ^{e}Sensient; ^{f}Ex. IFF AUBADE 1 containing β-ionone and other perfume ingredients with iodine values less than 10; ^{g}E. IFF AUBADE 2 not containing the β-lonone. | | | | |

The consumers were asked to rate key hedonic attributes on one of a 5, 7, or 10 point scale. Table 2 records these attributes and the scale employed. The consumer results obtained after the testing was finished were analysed for variance using ANOVA and paired t-testing, and the results of key hedonic attributes are recorded in Table 2 below which show a significant difference at 95 % confidence interval. Finally, Table 3 shows the results of how freshness was rated on a 10 point scale for the Control and Test Formulations across key consumer interventions. These results are significantly different at a 95 % confidence interval.

**Table 2**

| **Hedonic Attribute** | **Point Scale Used** | **Control** | **Test Formulation** |
|---|---|---|---|
| Fragrance Liking | 7 | 4.51 | 4.79 |
| Fragrance Intensity | 10 | 4.47 | 4.70 |
| Leaves a Smell of Cleanliness | 5 | 3.81 | 4.02 |
| Cleans Quickly | 5 | 4.11 | 4.20 |
| Fragrance Appeal 4 hours after Application | 7 | 4.36 | 4.44 |

**Table 3**

| **Consumer Intervention** | **Control Rating** | **Test Rating** | **Significantly Different at 95 % Confidence Interval** |
|---|---|---|---|
| Smell from Bottle | 7.7 | 7.6 | No |
| When used neat on a surface | 7.3 | 7.4 | No |
| When diluting | 7.4 | 7.4 | No |
| Whilst cleaning the floor | 7.3 | 7.4 | No |
| After the cleaner dried | 6.8 | 7.0 | Yes |
| When smelling the room after 4 hours | 5.5 | 5.9 | Yes |
| When smelling the room at the end of the day | 4.5 | 5.0 | Yes |
| When smelling the room the following day | 3.8 | 4.0 | Yes |

The results clearly show that the Test product containing the β-ionone pro-fragrance compound outperforms the control product across several hedonic attributes and at several key consumer intervention points for rating of freshness.

The choice of β-ionone as a pro-fragrance compound is by way of illustration and not by way of limitation.

### Example 2

These two experiments show that ozone is important for cyclocitral production, based on altering the ozone concentration and then measuring concentrations of ionone and cyclocitral and then calculating the ratio of cyclocitral/ionone which demonstrates that ozone increases the rate of production of cyclocitral.

### Experiment A

A β-ionone solution (10 % w/w in ethanol) was applied to a surface (20 microlitres) and was allowed to dry for one hour in a fume cupboard. The surface was then placed at the base of the ozone chamber. The ozone generator plug-in was turned on for the required time at regular intervals to achieve the desired ozone concentration inside the chamber (either 250 ppb or 350 ppb). After exposure of a surface to ozone for the desired time of 1 hour the surface was removed from the chamber and placed in a capped PVC container. The surface was left in the PVC container for 10 min and then an additional 20 min with an SPME fibre exposed to the head-space environment of the container. The headspace was measured by SPME-GCMS and the ratios of β-cyclocitral to β-ionone determined as follows:

| | |
|---|---|
| 250 ppb ozone | 0.72 |
| 350 ppb ozone | 2.67 |

It can be seen the higher concentration of ozone produces more conversion to the aroma compound β-cyclocitral over the same time frame. 250 ppb is higher than would normally be present in the atmosphere. Those levels are typically 50 to 100 ppb but the lower levels of ozone would still cause the reaction, albeit at a slower rate.

### Experiment B

This experiment was run like Experiment A, except that the ozone level used was 100 ppb and after 1,2,3,4 and 5 hours measurements of headspace were made and the ratios of β-cyclocitral / β-ionone were calculated from headspace concentration. The following ratios were determined:

| | |
|---|---|
| 1 hour | 0.43 |
| 2 hour | 0.95 |
| 3 hour | 1.87 |
| 4 hour | 7.00 |
| 5 hour | 11.00 |

As can be seen from the above results as time proceeds even at 100 ppb ozone in the chamber the β-ionone depletes and β-cyclocitral increases relative to this.

### Example 3

This example demonstrates that no β-cyclocitral is produced when ozone is absent from the atmosphere. A 0.3% solution of β-ionone in ethanol and a 0.3% solution of β-cyclocitral were prepared. 1 ml of the β-ionone was placed on a piece of filter paper (cellulose, 75 mm dia.) and left for 2 minutes for the ethanol to evaporate. The filter paper was then placed in a glovebox containing an ozone "scrubber" and an ozone monitor (Aeroqual series 200 fitted with a 0 to 0.5 ppm sensor). Throughout the experiment the ozone level inside the glovebox was below the detection limit of the monitor. The ozone scrubber comprised an air pump (Interpet AirVolution AV4), pumping air at a rate of 600 l/hr through 0.75 g/l sodium thiosulphate solution.

After 1 hour exposure the filter paper was sealed in a 500 ml glass jar that had been adapted such that a SPME fibre could be introduced via a septum in the lid. The headspace above the sample was analysed using a Carboxen/DVB/PDMS SPME fibre using a 30 minute exposure. The fibre was then analysed by GC-MS. The filter paper was then removed, placed in a 28 ml glass vial and 3 ml of ethanol added. The vial was rollered for two hours after which the free ethanol was removed and analysed by GC-MS. The β-cyclocitral in ethanol solution was also analysed to indicate the appropriate retention time. No β-cyclocitral was detected in either the headspace or on the filter paper. This shows that in the absence of ozone the β-ionone is not being converted to β-cyclocitral. Taken together with Example 2 it is reasonable to conclude that it is the ozone, rather than some other factor, that is facilitating the conversion when ozone is present. It is also a reasonable conclusion that the differences observed in example 1 are due to the same conversion of β-ionone to β-cyclocitral by the action of ozone from the atmosphere.

## Claims

1. A composition for treating a surface, the composition having a pH in the range 5 to 8 and comprising a pro-fragrance compound having an unsaturated bond and an iodine value greater than 100, that releases an aroma compound on ozonolysis of said unsaturated bond, wherein the remainder of the composition does not contain more than 1wt% compounds having an iodine value greater than 10 and further contains an aroma compound having an iodine value of less than 10, wherein the pro-fragrance compound is β-ionone and wherein:
- the pro-fragrance is present in an amount of 0.1 to 10 wt%;
- the composition comprises a surfactant and the total surfactant content is from 2 to 15 wt%;
- the composition is a hard surface cleaner.

2. The composition of claim 1, wherein the remainder of the composition does not contain more than 0.5wt% compounds having an iodine value greater than 10.

3. The composition of claim 1 or claim 2 wherein ozonolysis of the unsaturated bond in the pro-fragrance releases cyclocitral.

4. The composition of any preceding claim, wherein the pro-fragrance is encapsulated within a shell, optionally wherein the shell has deposition aid.

5. The composition of any preceding claim, wherein the composition comprises a hydrophobic oil having an octanol/water partition co-efficient (log P) greater than 3.

6. Use of a composition as claimed in any preceding claim for treating a surface whereby after application of the composition to the surface the β-ionone. pro-fragrance reacts with ozone in the atmosphere to produce fragrance changing levels of cyclocitral.

## Patentansprüche

1. Zusammensetzung zur Behandlung einer Oberfläche, wobei die Zusammensetzung einen pH-Wert im Bereich von 5 bis 8 aufweist und eine Duftstoffvorstufen-Verbindung mit einer ungesättigten Bindung und einer Iodzahl von mehr als 100 umfasst, die bei Ozonolyse der ungesättigten Bindung eine Aromaverbindung freisetzt, worin der Rest der Zusammensetzung nicht mehr als 1 Gew.-% Verbindungen mit einer Iodzahl von mehr als 10 enthält und ferner eine Aromaverbindung mit einer Iodzahl von weniger als 10 enthält, wobei die Duftstoffvorstufen-Verbindung β-Jonon ist, und wobei
- die Duftstoffvorstufe in einer Menge von 0,1 bis 10 Gew.-% vorliegt;
- die Zusammensetzung ein Tensid umfasst und der gesamte Tensidgehalt 2 bis 15 Gew.-% beträgt;
- die Zusammensetzung ein Reinigungsmittel für harte Oberflächen ist.

2. Zusammensetzung nach Anspruch 1, wobei der Rest der Zusammensetzung nicht mehr als 0,5 Gew.-% Verbindungen mit einer Iodzahl von mehr als 10 enthält.

3. Zusammensetzung nach Anspruch 1 oder Anspruch 2, wobei die Ozonolyse der ungesättigten Bindung in der Duftstoffvorstufe Cyclocitral freisetzt.

4. Zusammensetzung nach irgendeinem vorhergehenden Anspruch, wobei die Duftstoffvorstufe in einer Hülle eingekapselt ist, wobei die Hülle gegebenenfalls eine Abscheidungshilfe aufweist.

5. Zusammensetzung nach irgendeinem vorhergehenden Anspruch, wobei die Zusammensetzung ein hydrophobes Öl mit einem Octanol/Wasser-Verteilungskoeffizienten (log P) von mehr als 3 umfasst.

6. Verwendung einer Zusammensetzung, wie in irgendeinem vorhergehenden Anspruch beansprucht, zur Behandlung einer Oberfläche, wobei nach dem Aufbringen der Zusammensetzung auf die Oberfläche die Duftstoffvorstufe β-Ionon mit Ozon in der Atmosphäre unter Bildung von sich ändernden Duftstoffkonzentrationen von Cyclocitral reagiert.

## Revendications

1. Composition pour traiter une surface, la composition ayant un pH situé dans la plage allant de 5 à 8 et comprenant un composé précurseur de parfum ayant une liaison insaturée et un indice d'iode supérieur à 100, qui libère un composé aromatique lors de l'ozonolyse de ladite liaison insaturée, dans laquelle le reste de la composition ne contient pas plus de 1 % en poids de composés ayant un indice d'iode supérieur à 10, et contient en outre un composé aromatique ayant un indice d'iode inférieur à 10, dans laquelle le composé précurseur de parfum est la β-ionone et dans laquelle :
- le précurseur de parfum est présent en une quantité de 0,1 à 10 % en poids ;
- la composition comprend un tensioactif et la teneur totale en tensioactif est de 2 à 15 % en poids ;
- la composition est un nettoyant pour surfaces dures.

2. Composition selon la revendication 1, dans laquelle le reste de la composition ne contient pas plus de 0,5 % en poids de composés ayant un indice d'iode supérieur à 10.

3. Composition selon la revendication 1 ou la revendication 2, dans laquelle l'ozonolyse de la liaison insaturée dans le précurseur de partum libère du cyclocitral.

4. Composition selon l'une quelconque des revendications précédentes, dans laquelle le précurseur de parfum est encapsulé à l'intérieur d'une coque, éventuellement dans laquelle la coque a un auxiliaire de déposition.

5. Composition selon l'une quelconque des revendications précédentes, laquelle composition comprend une huile hydrophobe ayant un coefficient de séparation octanol/eau (logP) supérieur à 3.

6. Utilisation d'une composition de l'une quelconque des revendications précédentes pour traiter une surface, moyennant quoi, après application de la composition à la surface, le précurseur de parfum β-ionone réagit avec l'ozone dans l'atmosphère pour produire des niveaux changeants de parfum cyclocitral.
